# EUROPÄISCHE PATENTSCHRIFT

(11) **EP 1 028 796 B1**
(45) Veröffentlichungstag und Bekanntmachung des Hinweises auf die Patenterteilung: **02.05.2002**
(21) Anmeldenummer: 98962222.0
(22) Anmeldetag: 03.11.1998
(51) Int. Cl.: B01D 53/00

(54) **VERFAHREN UND VORRICHTUNG ZUR RÜCKGEWINNUNG VON GASEN**
METHOD AND DEVICE FOR RECOVERING GASES
PROCEDE ET DISPOSITIF DE RECUPERATION DE GAZ

(30) Priorität: 04.11.1997 DE 19749963; 09.10.1998 DE 19847950
(43) Veröffentlichungstag der Anmeldung: 23.08.2000
(73) Patentinhaber: Pneumatik Berlin Gmbh, 13088 Berlin (DE)
(72) Erfinder: STACH, Helmut, D-12439 Berlin (DE); BACHERT, Hans-Jürgen, D-15732 Schulzendorf (DE); WELKE, Hartmut, D-16356 Ahrensfelde (DE)
(74) Vertreter: Wehlan, Helmut, Dr.
(86) Internationale Anmeldenummer: DE9803260
(87) Internationale Veröffentlichungsnummer: WO9922845

(56) Entgegenhaltungen:
- EP-A- 0 284 227
- DE-A- 4 233 577
- DE-A- 19 549 271
- US-A- 5 417 742
- OTTEN, GAIL, FREY: 'Einsatzmöglichkeiten hydrophober Zeolithe in der Adsorptionstechnik' CHEM.-ING.-TECH. Bd. 64, Nr. 10, Seiten 915 - 925

## Beschreibung

Die vorliegende Erfindung betrifft ein Verfahren und eine Vorrichtung zur Rückgewinnung von Gasen nach den Ansprüchen 1 und 4. Anwendungsgebiete sind die in der Human- und Veterinärmedizin verwendeten Narkosegase sowie die Rückgewinnung von Gasen aus Abluft. Die Vorrichtung ermöglicht die Zwischenspeicherung der Gase in geeigneten Zeolithen und die vollständige Rückgewinnung der Gase aus den während der Anwendung entstehenden Gasgemischen durch thermisch induzierte Austreibung aus diesen Zeolithen.

Bekannt sind Vorrichtungen, mit deren Hilfe Gasgemische durch ihr Einleiten in definierte chemische Stoffe derart aufgespalten werden, daß die gewünschte Abtrennung durch zumeist chemische Reaktion erfolgt. In dieser Weise entstehen neue Verbindungen, die entweder in der vorliegenden Form entsorgt oder langzeitgelagert werden, in den meisten Fällen jedoch nur sehr schwer zurückzugewinnen sind. Beispiele für absorptive Gasreiniger, die auf dem Prinzip des Durchströmens einer geeigneten chemisch aktiven Flüssigkeit beruhen, sind sogenannte Scrubber, wie sie in der Halbleiterindustrie für hochtoxische Prozeßgase Verwendung finden oder auch verschiedene Trockenbettabsorber, deren aktive Komponenten für die verschiedensten abzutrennenden Gase optimiert werden können. Alle diese Anlagen weisen den gemeinsamen Nachteil auf, daß sie nicht fiir eine wirtschaftliche Rückgewinnung der in ihnen sorbierten Gase ausgelegt sind.

Es ist ebenfalls allgemein bekannt, daß neben der genannten Absorption in geeigneten Flüssigkeiten und Feststoffen, mikroporöse Festkörper wie Zeolithe, Aktivkohle u.a. bestimmte Stoffe unter Abgabe von Wärmeenergie adsorbieren und bei Aufnahme von Wärmeenergie desorbieren können. Ähnliche Prozesse laufen auch bei physikalischen Zustandsänderungen (z.B. Eis-Wasser-Dampf) ab.

Der Stand der Technik weist eine Vielzahl von Veröffentlichungen zur Sorption der verschiedensten Gase in Flüssigkeiten, festen Absorbern aber auch Zeolithen und anderen mikroporösen Festkörpern auf. Gasmaskenfilter absorbieren und adsorbieren Schadstoffe bis zum Erreichen einer Sättigungsgrenze ebenfalls vollständig, jenseits der, relativ leicht kontrollierbar, die Stoffe praktisch unbeeinflußt durchgelassen werden. Dies bedeutet, daß sich stets ein Grenzwert für die adsorbierten Gase - z.B. Narkosegase - einstellt, bei dem sich die Sorbentien und die Sorptive im Gleichgewicht befinden. Dieser Punkt ist im wesentlichen von Druck und Temperatur der Komponenten abhängig.

Die technischen, physikalischen und chemischen Voraussetzungen für eine möglichst hohe Sorptionskapazität, verbunden mit einer optimalen Regenerierbarkeit der Sorptionsanlagen sind in den Patentschriften DE-A-3731688, DE-A-3628858 und DD-A-239947 beschrieben.

In den Schriften DE-A-4003668 und DE-A-3713346 wird über die Entfernung von halogenierten Kohlenwasserstoffen mittels Zeolithen berichtet. In der Offenlegungsschrift DE-A-19549271 wird ein Verfahren zur Rückgewinnung von Gasen unterschiedlichen Dampfdrucks aus Gasgemischen beschrieben, bei dem siliciumreiche Zeolithe eingesetzt werden. Über das Si/Al-Verhältnis werden in dieser Schrift keine Angaben gemacht. Zeolithe eignen sich ebenfalls zum Entfernen von Stoffen aus wäßrigen Lösungen (DE-A-44 06766 und DE-A-19531933). In der letzten Zeit finden vor allem aluminiumarme und dealuminierte Zeolithe als Adsorptionsmittel Verwendung, wie der Patentschrift DE-A-19532500 zu entnehmen ist. Die Sorption von halogenierten Kohlenwasserstoffen an dealuminierten Zeolithen wird in der DE-A-4233577 beschrieben.

Die bekannten kommerziell genutzten Anordnungen weisen die Gemeinsamkeit auf, daß entweder die beim Sorptionsprozeß abgeschiedenen Stoffe dort gebunden bleiben und mit den Sorbentien gemeinsam. beispielsweise in geeigneten Verbrennungsanlagen, entsorgt oder auch auf chemischem Wege in relativ unschädliche Produkte umgewandelt und anschließend deponiert werden. So sind u.a. bestimmte Gasmasken mit zumeist aus modifizierter Aktivkohle bestehenden Sorbentien gefüllt.

Über die Rückgewinnung von Narkosegas mit absorbierendem Material wird im US-Patent US-A-3,592.191 berichtet. Dabei wird der Wasserdampf mittels eines hygroskopischen Materials gebunden.

Otten. Gail und Frey (Chem.-Ing.-Tech. 64 (1992) Nr. 10, 915-25) stellen einen dealuminierten Y-Zeolith mit einem SiO₂/Al₂O₃-Verhältnis von über 200 vor, der sich zur Abluftreinigung und Lösungsmittelrückgewinnung, z.B. von Toluol, eignet. Diese hochdealuminierten Zeolithe weisen allerdings noch eine gute Adsorptionsfähigkeit für polare Stoffe auf.

Im Patent EP-A-0 284227 sind eine Apparatur und ein Verfahren beschrieben, bei der Alumosilikate bis zu 15 Gew.-% von Narkosegasen mit relativ kleinen Moleküldurchmessern aufnehmen können. Bei dem dort vorgestellten Verfahren wird auch eine Rückgewinnung derart vorgeschlagen, daß ein erhitztes Trägergas den gefüllten Adsorber durchströmt, einen größeren Teil des adsorbierten Gases zur Desorption bringt und bei nachfolgender Abkühlung auf tiefe Temperaturen, die aus verdampfendem flüssigen Stickstoff gewonnen werden, in einem geeigneten Behälter wieder kondensieren und somit einer Wiederverwendung zugeführt werden können. Es werden Zeolithe mit einem SiO₂/Al₂O₃-Verhältnis größer 50 eingesetzt. Rückgewinnungsgrad liegt bei diesem Verfahren deutlich unter 50 %; eine Aussage über die Qualität der rückgewonnenen Gase wird nicht gegeben. Sevofluran wird nach diesem Verfahren nicht abgetrennt. Da die in diesem Patent verwendeten Alumosilikate jedoch einen relativ hohen Anteil an Aluminium aufweisen, das für seine katalytische Aktivität bei den in Frage stehenden Narkosegasen, die sämtlich halogenierte Kohlenwasserstoffe darstellen, bekannt ist, erscheint eine direkte Wiederverwendung wegen der zu erwartenden katalytischen Folgeprodukte nicht möglich. Die notwendigerweise hohen Temperaturen zur Desorption mittels heißen Trägergases verstärken diesen Effekt noch.

Die nahezu vollständige Rückgewinnung von Narkosegasen ist noch nicht beschrieben worden. Der Erfindung liegt die Aufgabe zugrunde, ein Verfahren und eine Vorrichtung zur Sorption vornehmlich gasförmiger Stoffe, beispielsweise Narkosegase, zur Verfügung zu stellen, die es gestatten, die nahezu vollständige Rückgewinnung dieser Stoffe beispielsweise aus der vom Patienten ausgeatmeten Luft zwecks Kostenersparnis oder zur Entlastung der Umwelt bei möglichst geringem Energieaufwand und minimaler Beeinflussung der Zusammensetzung der Narkosegase zu ermöglichen.

Diese Aufgabe wird durch das erfindungsgemäße Verfahren und die Vorrichtung nach den Ansprüchen 1 und 4 gelöst, indem unter Ausnutzung von in geeigneter Weise angepaßten Desorptionsprozessen einzelne Komponenten der Gemische oberhalb einer stoffspezifischen Temperatur in adsorbierenden Zeolithen gebunden werden, während andere Komponenten diese passieren. wobei man das sorbierte Gas mittels Erwärmen der sorbierenden Stoffe desorbiert, in einem anschließenden Kondensator verflüssigt und einer Wiederverwendung zuführt.

Es werden als Adsorptionsmittel Si-reiche Zeolithe eingesetzt, mit
a) einem extrem hohen Si/Al Verhältnis von größer 180:1 (entspricht einem SiO₂/Al₂O₃-Verhältnis von 360:1), was die katalytischen Reaktionen mit dem Narkosegas vermindert.
b) einer erheblich verringerten Adsorptionswärme. Die Größe der Adsorptionswärme bestimmt die Desorptionstemperatur, die möglichst niedrig sein sollte, um katalytische Reaktionen zu vermeiden.
c) einem Adsorptionsvermögen für alle gegenwärtig verwendeten Inhalationsanästhetika.
d) einer größeren Adsorptionskapazität für Narkosegase.
e) verformtem Zeolithrnaterial (Hohlkörper mit Abmaßen bis zu 10 mm), womit die Strömungseigenschaften wegen des größeren Sekundärporensystems verändert werden und damit die Adsorptionskapazität und die Kinetik der Adsorption positiv beeinflußt werden.
f) einem Porendurchmesser um 0,7 nm
g) hydrophoben und organophilen Charakter, die ein geringes oder kein elektrostatisches Feld in den Poren und Hohlräumen aufweisen

Überraschenderweise hat sich herausgestellt, daß die erfindungsgemäße Lösung eine katalytische Reaktion mit dem Narkosegas nahezu ausschließt und seine Wiederverwendung bzw. Neukonfektionierung ermöglicht.

Das erfindungsgemäße Verfahren und die Vorrichtung haben folgende Vorteile:
- keine oder nur geringe oder durch einfache Spülvorgänge zu beseitigende Adsorption von Luft, Lachgas, Kohlendioxid. Wasserdampf,
- großes Aufnahmevermögen für die bekannten Narkosegase, insbesondere Halothan, Isofluran, Sevofluran, Desfluran und Enfluran,
- möglichst geringe katalytische Reaktion mit fluorierten Kohlenwasserstoffen, um eine entweder direkte Wiederverwendbarkeit oder eine kostengünstige Aufarbeitung und Neukonfektionierung zu erreichen,
- energetisch günstiger Betrieb durch Verzicht auf Transportgase, deren Masse ein Vielfaches der rückzugewinnenden Gase beträgt,
- Verwendung eines Kühlsystems, das keine anderen Anschlüsse als elektrische benötigt.

Das Verfahren und die Vorrichtung ermöglichen die vollständige Rückgewinnung der entsprechenden Gase.

Die Vorrichtung zur Sorption und Rückgewinnung beinhaltet einen Adsorber 1 mit integrierten Heizelementen 2, in dem das zu entsorgende Gasgemisch durch geeignete gastechnische Verbindung mit dem vorhandenen Abluftsystem gesammelt wird. Über die Verbindungsleitung 3 und das Ventil 4 wird im Falle der Rückgewinnung der durch die Heizer 2 erwärmte, im Adsorber befindliche Zeolith mit Hilfe der Chemievakuumpumpe 9 bei geringem Druck desorbiert. Eventuelle geringe höhersiedende Komponenten des Gases, zumeist Wasser, werden im Vorabscheider 10 aufgefangen.

Die Vakuumpumpe dient nicht nur der Unterstützung der Desorption, sondern bewirkt vor allem eine Verringerung der thermischen Belastung des Desortionsprozesses. um eine Zersetzung der Inhalationsanästhetika zu verhindern. Die Chemievakuumpumpe stellt die Reinheit des kondensierten Produktes sicher und ermöglicht gute Ausbeuten. Der Vorabscheider trennt nicht nur das Wasser ab, sondern verringert eine mögliche Adsorption des in der Exspirationsluft im großen Überschuß im Vergleich zum Narkosegas vorhandenen Lachgases.

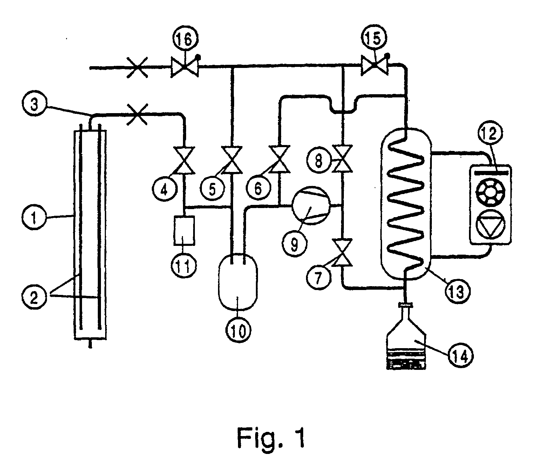

Der erforderliche Druck, der durch die Leistungsfähigkeit der Pumpe 9, die Temperatur der Heizelemente 2, die Masse des Zeoliths im Adsorber 1 und seine Beschaffenheit sowie den aktuellen Grad seiner Beladung mit einem gegebenen Narkosegas bestimmt ist, wird durch den Drucksensor 11 erfaßt und durch geeignete Stellung der Ventile 5 und 6 in Verbindung mit einer elektronischen Regelung so geführt, daß eine optimale Kondensation im Kondensator 13. dem das desorbierte Gas über das Ventil 7 zugeführt wird. möglich ist. Die erforderliche Temperatur des Kondensators 13 wird von einem Kühlaggregat 12 mit luftgekühlten Peltierelementen bereitgestellt und auf die je nach Narkosegas optimale Temperatur zwischen - 5 und + 10 C geregelt. Die Anordnung der Peltierkühlung (siehe Fig. 1) ist völlig unüblich, da keine direkte Kühlung der Kondensatorflächen, sondern eine Kühlung eines Kühlmittelkreislaufes erfolgt. Diese Anordnung wurde gewählt. um eine schnelle Kühlung zu erreichen und die Kühlmitteltemperatur den unterschiedlichen Dampfdrücken der verschiedenen Narkosegase anpassen zu können.

Die Rückschlagventile 15 und 16 gestatten die vollständige Abtrennung der Apparatur und der Druckregelung von der umgebenden Atmosphäre, so daß eine nahezu 100%ige Kondensation der desorbierten Gase möglich wird. Die Anordnung der Ventile entspricht einer Sicherheitsmaßnahme, da austretendes konzentriertes Narkosegas zu einer Gefährdung des Personals und der Umwelt führt. Daher wird auch die gesamte Anlage im Unterdruck betrieben (siehe Fig. 1), und es werden zwei Rückschlagventile verwendet. Der Drucksensor ist zwischen beiden Ventilen angebracht. Durch geeignete Stellung der Ventile 4 bis 8 ist es darüber hinaus möglich, etwaige Kondensatrückstände oder auch geringe Mengen Wasser, die aus vorhergehenden Prozessen stammen, mit Hilfe der Vakuumpumpe 9 zu verdampfen und somit das Gesamtsystem zu reinigen. Der Kondensatauffangbehälter 14 sammelt das rückgewonnene Narkosegas und kann zur Vermeidung von Verwechslungen der vom Hersteller des Narkosegases verwendete Behälter sein. Temperaturmeßvorrichtungen. Gasmengenregulierung und Steuerelektronik für Ventile, Temperaturen und Druck sind nicht dargestellt.

Die Vorrichtung ist vorzugsweise zur Abtrennung und Wiedergewinnung von Narkosegasen aus Abluftsystemen in der medizinischen Anästhesie vorgesehen.

Zu diesem Zweck können Adsorber 1 auch wechselseitig zur Sorption (Abscheidung) des Narkosegases oder mit Hilfe eines geeigneten Temperatur/Zeitzyklus zur Desorption bei Temperaturen bis maximal 150 C benutzt werden, wobei auf an sich bekannte Weise das adsorbierte Gas wieder ausgetrieben und bei geöffneten Ventilen 4 und 7 im Kondensator 13 kondensiert und im Kondensatbehälter 14 gelagert wird.

Nach Unterbrechung der externen Energiezufuhr ist die Vorrichtung bei dann geschlossenen Ventilen auf einen neuen Prozeßdurchlauf vorbereitet und kann bis auf Umgebungstemperatur abkühlen, wonach der Adsorber abgetrennt und wieder zur Sorption von Narkosegas verwendet sowie bei Bedarf nunmehr ein zweiter Adsorber desorbiert werden kann.

Die zur genannten Zwischenspeicherung verwendeten, mit Zeolith gefüllten Adsorber sind dabei nicht notwendig in die eigentliche Rückgewinnungsapparatur integriert. sondern frei beweglich einsetzbar, so daß eine Apparatur bei entsprechender Dimensionierung auch mit mehreren Adsorbern zusammenwirken kann.

Die beschriebene Vorrichtung kann stationär oder auch mobil, neben der Narkosegasrückgewinnung auch zur Wiedergewinnung von anderen entweder umweltschädigenden und/oder teuren Gasen verwendet werden.

Das Wesen der Erfindung liegt in einer Kombination bekannter Elemente und neuer Lösungswege, die sich gegenseitig beeinflussen und in ihrer neuen Gesamtwirkung einen Gebrauchsvorteil und den erstrebten Erfolg ergeben, der darin liegt, daß nunmehr eine vollständige Rückgewinnung der entsprechenden Gase möglich ist.

Der Vorabscheider (10) ist mit Material aus hydrophilem Zeolith gefüllt, dessen Porendurchmesser kleiner ist als der Durchmesser der in Frage Kommenden rückzügewinnenden base und der entweder außerhalb oder innenhalb der Apparatur thermisch unterstützt desorbiert werden kann.

Die Funktionsweise der Vorrichtung wird an einem Ausführungsbeispiel näher erläutert, ohne sie auf dieses Beispiel zu beschränken.

### Ausführungsbeispiel

Zum Zwecke der Nutzung in Verbindung mit einem Anästhesiegerät wird eine stationäre Vorrichtung beschrieben. Unter der Annahme, daß während eines Operationszyklus von maximal 8 h ein Narkosegasgemisch, beispielsweise aus Lachgas und Sauerstoff (2/1, Vol./Vol.) mit einem Durchsatz von 1,5 l/min, worin 6 Vol.-% Desfluran enthalten sind, eingesetzt wird, ist bei dem verwendeten speziellen Zeolith, z.B dealuminierte Faujasite. eine Masse von etwa 3 kg erforderlich, um die genannte Desfluranmenge von etwa 0.33 kg bei Zimmertemperatur im Adsorber 1 zu binden. Da diese Masse etwa der in einer üblichen in der Anästhesie zur Anwendung kommenden Desfluranflasche enthaltenen Menge entspricht und je nach angewendetem Anästhesieverfahren eine mehr oder weniger große Menge vom Patienten mittelfristig aufgenommen wird, kann der Adsorber etwa die Kapazität einer Desfluranflasche aufnehmen und wird nach dieser Annahme an die Leitung 3 der Vorrichtung zur Einleitung des Rückgewinnungsprozesses angeschlossen. Da alle Komponenten der Vorrichtung vakuumdicht ausgeführt sind, wird nach einseitigem Verschließen des Adsorbers der folgende Prozeß ablaufen (Figur 1):

Zunächst werden etwaige Rückstände des vorherigen Prozesses durch Anschalten der Pumpe 9 und Öffnen der Ventile 6 und 8 aus dem Kondensator 13, dem Kondensatauffangbehälter 14 und den Leitungen verdampft und über das Rückschlagventil 16 aus der Anlage entfernt. Nachdem das Kühlaggregat 12 den Kondensator 13 auf die erforderliche Kondensationstemperatur abgekühlt hat, werden die Ventile 6 und 8 geschlossen und die Ventile 4 und 7 geöffnet. Dadurch entsteht ein mit dem Drucksensor 11 auswertbares Vakuum im Adsorber 1, das dazu führt, daß die Desorption einsetzt und eine entsprechende Masse im Behälter 14 aufgefangen werden kann. Da die Geschwindigkeit der Kondensation auch vom Massentransport durch die Pumpe 9 und damit vom Narkosegaspartialdruck im Adsorber 1 abhängt, wird dieser Druck im weiteren Verlauf des Rückgewinnungsprozesses durch allmähliche Temperaturerhöhung des Zeoliths mit Hilfe der Heizer 2 solange konstant gehalten, bis die maximale Temperatur erreicht ist und der Partialdruck über dem Zeolith mit fortschreitender Desorption abnimmt. Das Ende des Prozesses ist erreicht. wenn trotz hoher Temperatur der Druck im Adsorber 1 über längere Zeit bei etwa 10 mbar auch ohne die mit Hilfe des Drucksensors 11 geregelte Pumpe 9 verharrt. Die im Adsorber 1 verbleibende Restmasse Narkosegas liegt unter diesen Bedingungen bei unter 1 g, also etwa 0,1 Gew.-%.

Durchschnittlich werden nahezu 100 % des vom Patienten emittierten Narkosegases adsorbiert und über 90 % zurückgewonnen. Je nachdem, ob der Anästhesist höhere oder niedrigere Narkosegaskonzentrationen anwendet, ist allerdings der Anteil des während einer Narkose vom Patienten emittierten und damit adsorbierbaren Narkosegases unterschiedlich. In jedem hier untersuchten Fall lag jedoch die Rückgewinnungsrate, gemessen am Gesamteinsatz des Narkosegase, bei über 60 % und damit doppelt so hoch wie bei Alumosilikaten. Gaschromatographische Untersuchungen zur Reinheit der rückgewonnenen Gase ergaben Reinheiten zwischen 98,0 und 99,7 %. Die Resultate zeigten, daß eher noch nicht optimierte Gasführungselemente als Hauptkomponenten für die geringen Restverunreinigungen verantwortlich sind.

Bei Einsatz von Isofluran ergibt sich wegen der dort typischerweise angewendeten geringeren Konzentration bei gleicher Adsorptionskapazität eine etwa 6-fach höhere Einsatzdauer der Adsorber bis zur Desorption.

### Bezugszeichenliste

- 1: Adsorber
- 2: Heizer
- 3: Gasleitung
- 4: Ventil
- 5: Ventil
- 6: Ventil
- 7: Ventil
- 8: Ventil
- 9: Chemievakuumpumpe
- 10: Vorabscheider
- 11: Drucksensor
- 12: Peltier-Kühlaggregat
- 13: Kondensator
- 14: Kondensatauffangbehälter
- 15: Rückschlagventil
- 16: Rückschlagventil

## Patentansprüche

1. Verfahren zur Entfernung und Rückgewinnung von Gasen unterschiedlichen Dampfdrucks aus Gasgemischen, bei dem einzelne Komponenten der Gemische oberhalb einer stoffspezifischen Temperatur in adsorbierenden Zeolithen gebunden werden, während andere Komponenten diese passieren, wobei man das sorbierte Gas mittels Erwärmen der sorbierenden Stoffe desorbiert, anschließend in einem Kondensator verflüssigt und einer Wiederverwendung zuführt, **dadurch gekennzeichnet, daß** die Zeolithe Si-reiche Zeolithe sind, die ein Si/Al-Verhältnis größer 180 und einen Porendurchmessem um 0,7 nm besitzen.

2. Verfahren nach Anspruch 1, **dadurch gekennzeichnet, daß** die Zeolithe hydrophoben und organophilen Charakter besitzen und ein geringes oder kein elektrostatisches Feld in den Poren und Hohlräumen aufweisen.

3. Verfahren nach Anspruch 1 und 2, **dadurch gekennzeichnet, daß** die Zeolithe aus verformtem Zeolithmaterial, in Form von Hohlkörpern bis zu 10 mm, bestehen.

4. Vorrichtung zur Durchführung des Verfahrens nach Anspruch 1 bis 3, beinhaltend einen Adsorber (1) zur Sammlung des emittierten Gases, der über eine Leitung (3) und Ventile (4 und 7) mit einer Chemievakuumpumpe (9) verbunden ist, die ihrerseits das durch Unterdruck und Erwärmung mittels der Heizer (2) desorbierte Gas in den von einem Kühlaggregat (12) geeignet temperierten Kondensator (13) leitet, wodurch sich das Gas verflüssigt und im Kondensatauffangbehälter (14) gesammelt wird, **dadurch gekennzeichnet, daß** als Adsorber Si-reiche Zeolithe eingesetzt werden, die ein Si/Al-Verhältnis größer 180 und einen Porendurchmesser um 0,7 nm besitzen.

5. Vorrichtung nach Anspruch 4, **dadurch gekennzeichnet, daß** ein Vorabscheider (10) mit Material aus hydrophilem Zeolith gefüllt ist, dessen Porendurchmesser kleiner ist als der Durchmesser der in Frage kommenden rückzugewinnenden Gase und der entweder außerhalb oder innerhalb der Apparatur thermisch unterstützt desorbiert werden kann.

6. Vorrichtung nach Anspruch 4 und 5, **dadurch gekennzeichnet, daß** mittels des durch einen Drucksensor (11) gesteuerten Ventils (5) und der geeigneten Anordnung der Rückschlagventile (15) und (16) nicht kondensiertes Gas wieder zum Kondensator zurückgeführt wird.

7. Vorrichtung nach Anspruch 4, **dadurch gekennzeichnet, daß** es sich bei dem Kühlaggregat (12) um einen Peltierkühler handelt.

8. Verwendung des Verfahrens nach Anspruch 1 bis 3 und der Vorrichtung nach Anspruch 4 bis 7 zur Rückgewinnung von Anästhesiegasen.

## Claims

1. Method for removing and recovering gases having different steam pressures from gas mixtures, wherein individual components from the mixture are bound above a substance-specific temperature in absorbent zeolites while other components pass said zeolites, the adsorbed gas being desorbed by heating the adsorbing substances, then liquified in a condenser and lead to reuse, **characterized in that** the zeolites are Si-rich zeolites having a Si/Al ratio greater than 180 and a pore diameter of approximately 0.7 nm.

2. Method according to claim 1, **characterized in that** the zeolites possess a hydrophobic and organophilic character and have a minor or no electrostatic field in the pores and hollow spaces.

3. Method according to claim 1 and 2 **characterized in that** the zeolites are comprised of deformed zeolite material in the form of hollow bodies up to 10 mm.

4. Apparatus for carrying out the method according to claims 1 to 3, including an adsorber (1) for collecting the emitted gas, said adsorber being connected via a connection line (3) and valves (4 and 7) to a chemical vacuum pump (9) which in turn feeds the gas which has been desorbed through underpressure and heating up by the heating elements (2) to a condenser (13) kept at a suitable temperature by a cooling aggregate (12), whereby the gas is liquified and collected in a condensate reservoir (14), **characterized in that** Si-rich zeolites are used for the adsorber, which have a Si/Al ratio greater than 180 and a pore diameter of approximately 0.7 nm.

5. Apparatus according to claim 4, **characterized in that** a prefractionator (10) is filled with material of hydrophilic zeolite, the pore diameter of which is smaller than the diameter of the gases to be considered for recovery and which can be desorbed with thermal assistance either without or within the system.

6. Apparatus according to claims 4 and 5, **characterized in that** by means of the valve (5) controlled by a pressure sensor (11) and a suitable arrangement of the return valves (15 and 16), gas which has not condensed is fed back to the condenser.

7. Apparatus according to claim 4, **characterized in that** a Peltier cooler is used for the cooling aggregate (12).

8. Use of the method according to claims 1 to 3 and the apparatus according to claims 4 to 7 for recovering anesthetic gases.

## Revendications

1. Procédé d'élimination et de récupération des gaz à des pressions de vapeur différentes et provenant de mélanges gazeux, par lequel les divers composants des mélanges sont liés dans des zéolithes absorbants au-dessus d'une température spécifique à la substance, tandis que d'autres composants traversent ceux-ci, le gaz absorbé étant désorbé par chauffage des substances absorbantes, puis liquéfié dans un condensateur et ramené vers une réutilisation, **caractérisé en ce que** les zéolithes sont des zéolithes riches en Si et ont un rapport Si/Al supérieur à 180 et un diamètre des pores d'environ 0,7 nm.

2. Procédé selon la revendication 1, **caractérisé en ce que** les zéolithes ont un caractère hydrophobe et organophile et présentent un champ électrostatique faible, voire nul, dans les pores et cavités.

3. Procédé selon les revendications 1 et 2, **caractérisé en ce que** les zéolithes sont constitués par un matériau de zéolithe déformé, sous la forme de corps creux pouvant atteindre jusqu'à 10 mm.

4. Dispositif pour mettre en oeuvre le procédé selon les revendications 1 à 3, comprenant un adsorbeur (1) pour recueillir le gaz émis, ledit adsorbeur étant lié à travers une conduite (3) et des soupapes (4 et 7) à une pompe à vide (9) pour produits chimiques qui, quant à elle, conduit le gaz désorbé en raison de la dépressuration et du chauffage occasionnés par les éléments de chauffage (2) dans un condensateur (13) tempéré de manière appropriée par un groupe frigorifique (12), ce qui entraîne la liquéfaction du gaz et son recueil dans le réservoir collecteur (14), **caractérisé en ce que** des zéolithes riches en Si sont utilisés en tant qu'adsorbeurs ayant un rapport Si/Al supérieur à 180 et un diamètre des pores d'environ 0,7 nm.

5. Dispositif selon la revendication 4, **caractérisé en ce qu'**un préfractionnateur (10) est rempli d'un matériau en zéolithe hydrophile dont le diamètre des pores est plus petit que le diamètre des gaz en question à récupérer, et qui peut être désorbé soit à l'intérieur, soit à l'extérieur de l'appareillage moyennant un soutien thermique.

6. Dispositif selon les revendications 4 et 5, **caractérisé en ce qu'**au moyen d'une soupape (5) commandée par un palpeur de pression (11) et par l'arrangement approprié des soupapes anti-retour (15) et (16), le gaz non condensé est reconduit vers le condensateur.

7. Dispositif selon la revendication 4, **caractérisé en ce que** pour le groupe frigorifique (12) il s'agit d'un groupe Peltier.

8. Utilisation du procédé selon les revendications 1 à 3 et du dispositif selon les revendications 4 à 7 pour la récupération des gaz d'anesthésie.
